Europäisches Patentamt

European Patent Office

Office européen des brevets

(11) Veröffentlichungsnummer: **0 487 027 A1**

## EUROPÄISCHE PATENTANMELDUNG

(21) Anmeldenummer: **91119721.8**

(22) Anmeldetag: **19.11.91**

(51) Int. Cl.5: **A61F  4/00**

(30) Priorität: **22.11.90 DE 4037049**

(43) Veröffentlichungstag der Anmeldung:
**27.05.92 Patentblatt  92/22**

(84) Benannte Vertragsstaaten:
**AT BE CH DE DK ES FR GB GR IT LI LU NL SE**

(71) Anmelder: **Schmitt, Walter, Dipl-Ing.**
**Königshügel 27**
**W-5100 Aachen(DE)**
Anmelder: **Zang, Werner, Prof.Dr.-Ing.**
**Weiherstrasse 14**
**W-5120 Herzogenrath(DE)**

(72) Erfinder: **Schmitt, Walter, Dipl-Ing.**
**Königshügel 27**
**W-5100 Aachen(DE)**
Erfinder: **Zang, Werner, Prof.Dr.-Ing.**
**Weiherstrasse 14**
**W-5120 Herzogenrath(DE)**

(74) Vertreter: **Bauer, Hubert, Dipl.-Ing. et al**
**Am Keilbusch 4**
**W-5100 Aachen(DE)**

(54) **Bedienungsvorrichtung für Körperbehinderte Personen.**

(57) Für mit Hilfe einer Mikrocomputer-Steuereinheit bedienbare Geräte wird eine Schalteinrichtung vorgeschlagen, die aus einer Platte (11) mit einem Tasterfeld (12) besteht. Die Platte (11) ist beispielsweise als Gaumenplatte intraoral so positionierbar, daß sich das Tasterfeld (12) mit der Zunge betätigen läßt. Auf diese Weise ausgelöste Signale werden auf ein Interface übertragen, mit dem diverse Geräte zu bedienen oder zu steuern sind.

Fig.1

Die Erfindung betrifft eine Bedienungsvorrichtung für mit Hilfe einer Mikrocomputer-Steuereinheit bedienbare Geräte. Dabei reagiert die Steuereinheit auf durch eine Schalteinrichtung auslösbare Signale.

Eine derartige, aus der CH 660 956 bekannte Vorrichtung versetzt körperbehinderte Personen, wie beispielsweise Tetraplegiker, die nur mit dem Mund oder der Zunge arbeiten können, in die Lage, ohne fremde Mithilfe technische Mittel in der Alltagsumgebung, wie z.B. Fernsehgerät, Radio, Telefon oder Alarmeinrichtungen u.s.w., auf einfache Weise mit einem einzigen Kontakt selber zu steuern. Dazu ist ein elektronisches Steuergerät vorgesehen, mit welchem der mit dem Mund oder der Zunge betätigbare Kontakt über eine Leitung verbunden ist. Das Steuergerät steht über weitere Leitungen mit den zu bedienenden Einrichtungen und einem Monitor in Verbindung. Sämtliche Steuerungen werden über den einzigen Mund- oder Zungenkontakt ausgelöst. Bei einer ersten Betätigung des beispielsweise als Zungensensor ausgebildeten Kontaktes wird ein als Monitor dienendes Fernsehgerät über einen Infrarot-Sender eingeschaltet. Auf dem Bildschirm des Fernsehgerätes erscheint zunächst eine Menü-Übersicht, wobei ein laufender Pfeil von Anzeige zu Anzeige wandert. Bei erneuter Betätigung des Kontaktes wird in Abhängigkeit von der jeweiligen Pfeilposition auf dem Bildschirm ein der jeweiligen Anzeige entsprechendes Unterprogramm aufgerufen, aus dem wiederum mit Hilfe eines laufenden Pfeiles ein Unterprogramm durch erneute Kontaktbetätigung auswählbar ist.

Die bekannte Vorrichtung erlaubt zwar, durch einen einzigen Kontakt eine Vielzahl von Funktionen eines Fernsehgeräts und anderer technischer Geräte in der Alltagsumgebung eines Patienten zu steuern, hat jedoch den Nachteil, daß zur Steuerung einer bestimmten Einzelfunktion unter Umständen eine größere Anzahl feinanderfolgender Kontaktbetätigungen erforderlich ist. Soll der Patient beispielsweise mit Hilfe des einzigen Kontaktes einen an sich durch eine Tastatur mit regelmäßig über 100 Funktionstasten ausgestatteten Computer bedienen, so ließe sich zwar die Tastatur des Computers auf einem gesonderten Bildschirm darstellen. Die einzelnen Funktionstasten könnten gleichfalls mit einem laufenden Pfeil abgegriffen werden. Der Patient könnte dann jeweils bei entsprechender Pfeilposition durch Betätigung des einzigen Kontaktes die betreffende Taste in Funktion bringen. Diese Arbeitsweise wäre jedoch außerordentlich zeitaufwendig, da zur Auslösung der Funktion einer Taste der Pfeil jeweils die insgesamt dargestellten Tasten nacheinander abgreifen müßte.

Ungeachtet der bekannten Vorrichtung ziehen es Tetraplegiker daher vor, zur Bedienung eines Computers dessen Tastatur mit einem im Mund gehaltenen Stift zu betätigen. Diese Betätigungsart ist für den Patienten nicht nur außerordentlich anstrengend, sondern führt neben Haltungsschäden auch zu Fehlstellungen der den Stift krampfhaft festhaltenden Zähne.

Der Erfindung liegt die Aufgabe zugrunde, eine Bedienungsvorrichtung der eingangs beschriebenen Art so auszubilden, daß sich damit von einem Patienten nicht nur eine Vielzahl unterschiedlicher Geräte aktivieren läßt, sondern auch Einzelgeräte mit einer Vielzahl Funktionstasten, wie sie z.B. Computer aufweisen, effizient bedienen lassen, wobei insbesondere auch anstrengende und zu Haltungsschäden führende Relativbewegungen zwischen dem Patienten und der Schalteinrichtung gänzlich unterbleiben können.

Zur Lösung dieser Aufgabe wird von einer Bedienungsvorrichtung der im Oberbegriff des Anspruchs 1 genannten gattungsgemäßen Art ausgegangen, welche erfindungsgemäß die im kennzeichnenden Teil desselben angegebenen Merkmale aufweist.

Das erfindungsgemäße Tasterfeld ermöglicht es, mit der Schalteinrichtung in schneller Folge oder auch gleichzeitig unterschiedliche Signale auszulösen, welche die Mikrocomputer-Steuereinheit entsprechend umsetzt, um die verschiedenen Geräte zu aktivieren oder auch ein Gerät, wie z.B. die umfangreiche Tastatur eines Computers, vollständig bedienen zu können. Durch die intraorale Positionierung der als Tasterfeld ausgebildeten Schalteinrichtung bedarf es lediglich einer relativ einfach erlernbaren Zungenfertigkeit, wobei die hohe Sensibilität der Zunge nutzbar gemacht wird, um die Positionen der einzelnen Taster schnell und zuverlässig zu lokalisieren. Sobald sich der Patient mit den Funktionszuweisungen der einzelnen Taster hinreichend vertraut gemacht hat, gelangt er durch Übung zu einer Abspeicherung der zur Auslösung bestimmter Signale erforderlichen Zungenstellung in seinem Unterbewußtsein, vergleichbar der Fertigkeit, mit welcher nichtbehinderte Personen beispielsweise die Tastatur einer Schreibmaschine, eines Musikinstruments oder dergleichen bedienen können.

Das Tasterfeld kann nach Ausgestaltungen der Erfindung in eine Aufnahmeplatte integriert sein, die, wie beispielsweise eine Gaumenplatte, eine Teilfläche der Mundhöhlenbegrenzung abdeckt, oder auch in einen Aufnahmestreifen integriert sein, der eine Teilfläche einer Zahninnenseite abdeckt. Bei jeder dieser Ausbildungen und Anordnungen lassen sich die einzelnen Taster so positionieren, daß sie für die Zunge leicht erreichbar sind.

Um zur Tasterbetätigung der Zunge keinen Kraftaufwand abzuverlangen, sieht eine besonders vorteilhafte Ausgestaltung der Erfindung vor, das Tasterfeld aus auf Annäherung der Zunge kontinuierlich oder diskontinuierlich reagierenden Sensoren zu bilden, die in zur Zunge hin offene Bohrungen der Aufnahmeplatte oder des -streifens eingelassen sind. Bestehen die Sensoren beispielsweise jeweils aus einer Reflexionsinfrarotlichtschranke, läßt sich diese durch die Zungenannäherung zur Auslösung eines Signals veranlassen, ohne daß es eines Zungendruckes auf den Sensor bedarf.

Die Anzahl der Taster läßt sich beispielsweise auf elf über eine Gaumenplatte verteilte Sensoren beschränken, um dennoch ein Vielfaches von zehn unterschiedlichen Signalen aulösen zu können. Dazu sieht eine Ausgestaltung der Erfindung vor, den Abstand benachbarter Sensoren so zu bemessen, daß mit der Zunge wahlweise nacheinander an einzelnen oder auch zeitgleich an mehreren Sensoren Signaländerungen auslösbar sind. Dabei reagiert die Mikrocomputer-Steuereinheit jeweils unterschiedlich auf die Signale der einzelnen Sensoren und ebenso unterschiedlich auf geeignete Kombinationen.

Es besteht darüber hinaus die Möglichkeit, die Sensoren in Bohrungen des Sensorenträgers mit einem so großen Abstand vom Öffnungsquerschnitt der Bohrungen zu plazieren, daß die Eindringtiefe der Zunge in die Bohrungen variierbar ist. Damit läßt sich eine analoge Steuerung als Funktion der Zungeneindringtiefe in die Bohrungen bewerkstelligen.

Die aus dem Tasterfeld gebildete Schalteinrichtung kann über ein Kabel mit der Mikrocomputer-Steuereinheit verbunden werden. Es besteht aber auch die Möglichkeit, die Signalübertragung auf die Mikrocomputer-Steuereinheit telemetrisch zu bewirken.

Bei Verwendung einer Kabelverbindung erfolgt die Energieversorgung der Sensoren gleichfalls über das Kabel. Bei einer telemetrischen Signalübertragung besteht die Möglichkeit, die erforderliche Energiequelle für die Sensoren ebenso wie diese in das Aufnahmeorgan des Tasterfeldes zu integrieren und aufladbar auszubilden. Es besteht aber stattdessen auch die Möglichkeit, eine drahtlose, z.B. induktive Energieversorung von außen vorzusehen.

Als Aufnahmeorgan des Tasterfeldes bietet sich beispielsweise eine aus Dental-Kunststoff hergestellte Gaumen- oder Sublingualplatte nach Art einer Zahnprothese an. Zu deren Befestigung lassen sich die in der zahnärztlichen Prothetik üblichen Retentionselemente verwenden.

Eine Mikrocomputer-gesteuerte Signalaufbereitung in der Steuereinheit paßt die Sensoranordnung an anwendungsorientierte Bedienungsprogramme an. Die Steuereinheit hat dazu vorzugsweise folgende Schnittstellen:

- Schaltausgänge zur digitalen Fernbedienungsmöglichkeit mit potentialfreien Kontakten, beispielsweise in Form von Halbleiterschaltern;
- Analogausgänge -10V bis +10V mit z.B. 8-Bit-Auflösung;
- ein z.B. PC-kompatibles Tastatur-Interface, das die Simulation einer PC-Tastatur mit dem intrakorporalen Sensorfeld ermöglicht;
- Computer-Mouse-Emulation.

In der Zeichnung ist ein Ausführungsbeispiel einer erfindungsgemäßen Bedienungsvorrichtung schematisch dargestellt. Es zeigen:

Fig. 1   eine Gaumenplatte mit einem Sensorfeld;

Fig. 2   einen einzelnen Sensor in vergrößertem Maßstab;

Fig. 3   eine Seitenansicht des Sensors gemäß Fig. 2 bei angenäherter Zunge;

Fig. 4   eine Mikrocomputer-Steuereinheit, angeschlossen an drei unterschiedliche Geräte, als Blockschaltbild;;

Fig. 5   Sensoren, Eingabe-Port, Mikroprozessor und Ausgabe-Port als Blockschaltbild.

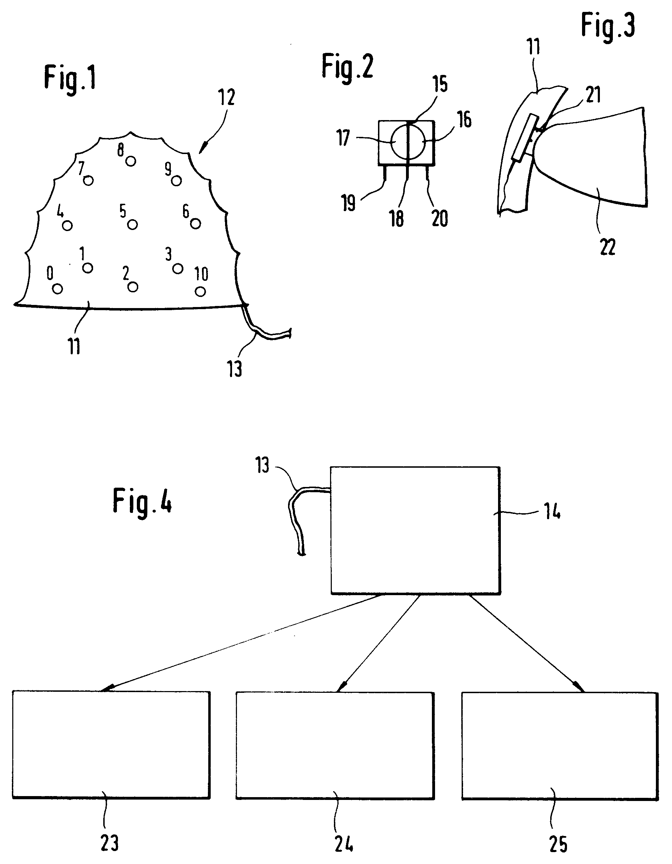

In Fig. 1 sind Sensoren 0 bis 10 über eine Gaumenplatte 11 verteilt angeordnet und bilden zungenseitig ein Tasterfeld 12. Die Gaumenplatte 11 und das Tasterfeld 12 stellen eine Schalteinrichtung dar, die sich nach Art einer Zahnprothese intraoral positionieren läßt. Über ein Kabel 13 ist jeder der Sensoren 0 bis 10 mit einer in Fig. 4 dargestellten Mikrocomputer-Steuereinheit 14 verbunden.

Wie Fig. 2 zeigt, weist jeder als Reflexionsinfrarotlichtschranke 15 ausgebildeter Sensor 0 bis 10 eine Sendediode 16 und eine Empfangsdiode 17 auf, an welche Leitungen 18, 19 und 20 angeschlossen sind, die das Kabel 13 bündelt.

Die Sensoren 0 bis 10 sind gemäß Fig. 3 jeweils in eine Bohrung 21 der aus Dental-Kunststoff gebildeten, abgebrochen dargestellten Gaumenplatte 11 so tief eingelassen, daß sie auf die Annäherung einer Zunge 22 noch reagieren.

Je nach Betätigung der Sensoren 0 bis 10 erhält die Mikrocomputer-Steuereinheit 14 unterschiedliche Signale und aktiviert wahlweise einen Computer 23, ein Telefon 24 oder irgendeine Haustechnik 25.

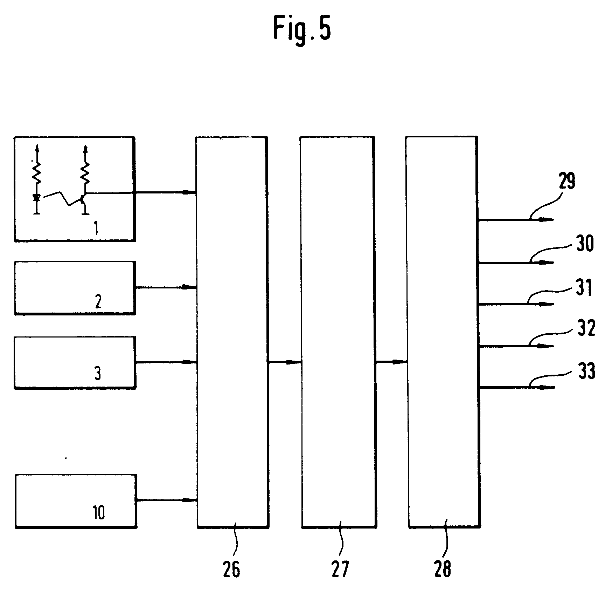

Wie das Blockschaltbild in Fig. 5 veranschaulicht, umfaßt die Mikrocomputer-Steuereinheit 14 einen an die Sensoren 0 bis 10 angeschlossenes Eingabe-Port 26, einen diesem nachgeschalteten Mikroprozessor 27 und einen Ausgabe-Port 28, woran über Leitungen 29 bis 33 nicht dargestellte Geräte, wie Telefon, Computer-Tastatur, Computer-Maus, Rollstuhl und diverse Haustechnik, angeschlossen sind.

**Patentansprüche**

1. Bedienungsvorrichtung für mit Hilfe einer Mikrocomputer-Steuereinheit aktivierbare Geräte, wobei die Steuereinheit auf durch eine Schalteinrichtung auslösbare Signale reagiert, dadurch gekennzeichnet, daß die Schalteinrichtung ein intraoral positionierbares, mit der Zunge (22) betätigbares Tasterfeld (12) umfaßt.

2. Bedienungsvorrichtung nach Anspruch 1, dadurch gekennzeichnet, daß das Tasterfeld (12) in eine eine Teilfläche der Mundhöhlenbegrenzung abdeckende Aufnahmeplatte (11) oder in einen eine Teilfläche einer Zahnreiheninnenseite abdeckenden Aufnahmestreifen integriert ist.

3. Bedienungsvorrichtung nach Anspruch 1 oder 2, dadurch gekennzeichnet, daß das Tasterfeld (12) aus auf Annäherung der Zunge (22) kontinuierlich oder diskontinuierlich reagierenden Sensoren (0 bis 10) besteht, die in zur Zunge (22) hin offene Bohrungen (21) der Aufnahmeplatte (11) oder des -streifens eingelassen sind.

4. Bedienungsvorrichtung nach mindestens einem der Ansprüche 1 bis 3, dadurch gekennzeichnet, daß durch den Abstand benachbarter Sensoren (0 bis 10) Signaländerungen wahlweise nacheinander an einzelnen oder zeitgleich an mehreren Sensoren (0 bis 10) auslösbar sind.

Fig.1

Fig.2

Fig.3

Fig.4

# Fig.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | WO-A-9 007 249 (ZOFCOM, INC.) | 1,3,4 | A61F4/00 |
| Y | * Zusammenfassung; Abbildungen * | 2 | |
| | --- | | |
| X | US-A-4 605 927 (KATZ ET AL.) | 1,3,4 | |
| | * Spalte 4, Zeile 65 - Spalte 5, Zeile 5 * | | |
| | --- | | |
| X | GB-A-2 094 949 (BRAMLEY) | 1 | |
| Y | * Zusammenfassung; Abbildungen 1-6 * | 2 | |
| | --- | | |
| A | FR-A-2 395 559 (INSTITUT NATIONAL DE LA SANTE ET OE LA RECHERCHE MEDICALE) * Seite 7, Zeile 38 - Seite 8, Zeile 8; Abbildungen * | 1 | |
| | --- | | |
| D,A | CH-A-660 956 (ROUX) | | |
| | ----- | | |

**RECHERCHIERTE SACHGEBIETE (Int. Cl.5 )**

A61F

Der vorliegende Recherchenbericht wurde für alle Patentansprüche erstellt

| Recherchenort | Abschlußdatum der Recherche | Prüfer |
|---|---|---|
| OEN HAAG | 05 FEBRUAR 1992 | SANCHEZ Y SANCHEZ J. |